# EUROPEAN PATENT APPLICATION

(11) **EP 4 696 245 A1**
(43) Date of publication of application: **18.02.2026**
(21) Application number: 25762369.4
(22) Date of filing: 26.02.2025
(51) Int. Cl.: A61B 8/08, A61B 8/00, A61N 7/00

(54) **FOCUSED ULTRASOUND PROCESSING SYSTEM FOR OPENING AND MONITORING BLOOD-BRAIN BARRIER AND ULTRASOUND CONTROL METHOD OF FOCUSED ULTRASOUND PROCESSING SYSTEM FOR OPENING AND MONITORING BLOOD-BRAIN BARRIER**

(30) Priority: 27.02.2024 KR 20240028056
(71) Applicant: Neumous Inc., Cheongju-si, Chungcheongbuk-do 28160 (KR)
(72) Inventor: PARK, Ju Young, Gyeonggi-do 13643 (KR); PARK, Chan Yuk, Seoul 03668 (KR)
(74) Representative: Schmitt-Nilson Schraud Waibel Wohlfrom Patentanwälte Partnerschaft mbB
(86) International application number: PCT/KR2025/002681
(87) International publication number: WO 2025/183452

(57) **Abstract**

A focused ultrasound processing system for opening and monitoring a blood-brain barrier (BBB) according to an embodiment of the present disclosure may include at least one piezoelectric element consisting of a single structure and a drive module that provides an electrical signal such that ultrasonic energy having at least one resonance frequency among a plurality of resonance frequencies is output by the at least one piezoelectric element.

## Description

### [TECHNICAL FIELD]

The present disclosure relates to a focused ultrasound processing system for opening and monitoring a blood-brain barrier and an ultrasound control method of the focused ultrasound processing system for opening and monitoring the blood-brain barrier.

The present disclosure is derived from research conducted as part of Drug Delivery Therapy Technology Development Project of the Ministry of Health and Welfare (Project Unique No.: 1465040354, Project No.: HI23C0344000023, Research Project Name: Development of Attachable Ultrasound System Combined with Drug Carrier for Brain Drug Delivery, Project Management Agency: Korea Health Industry Development Institute, Project Implementing Agency: Neumous Co., Ltd., Research Period: 2023.04.01 ~ 2027.12.31, Contribution Rate: 50%), and Startup Growth Technology R&D Project of the Ministry of Small and Medium Business and Startups (Project Unique No.: 1425179650, Project No.: 00261874, Research Project Name:Development of Patient-specific Multi-channel Focused Ultrasound System for Opening Blood-Brain Barrier, Project Management Agency: Korea SMEs Technology Information Promotion Agency, Project Implementing Agency: Neumous Co., Ltd., Research Period: 2023.06.01 ~ 2026.05.31, Contribution Rate: 50%). Meanwhile, there is no property interest of the Korean government in any aspect of the present disclosure.

### [BACKGROUND ART]

A blood-brain barrier (BBB) refers to a physiological barrier that exists within cerebral blood vessels to separate and protect a brain and a central nervous system. This barrier operates as a separator between nerve cells inside the brain and blood.

The inability of treatments for brain diseases to penetrate deep enough into the brain due to the BBB is a significant problem associated with many neurological and medical challenges. In other words, while serving to separate blood and brain tissues to keep the brain protected and safe, the BBB also makes effective delivery of brain disease therapeutics difficult.

Nowadays, various technologies are developed and studied to deliver brain disease treatments through the BBB, but there are various limitations such as limited or temporary effects, safety issues, or difficulties in accurate targeting.

Accordingly, there is need for a technology capable of not only efficiently and safely controlling the opening of the BBB, but also monitoring the entire processes to overcome the above-mentioned issues and effectively delivering the brain disease treatments into a brain.

### [DETAILED DESCRIPTION OF THE INVENTION]

### [TECHINICAL PROBLEM]

The technical problem to be solved by the present disclosure is to open BBB by outputting (or irradiating) ultrasonic energy having one of resonance frequencies of a plurality of ultrasonic piezoelectric elements.

Moreover, the technical problem to be solved by the present disclosure is to monitor in real time the opening degree of BBB (or the location and behavior of acoustic cavitation-inducing agents) and the focusing location (or a location in a brain where the BBB is opened) of ultrasonic energy by receiving an acoustic cavitation signal from an object to which ultrasonic energy is emitted (or irradiated).

Furthermore, the technical problem to be solved by the present disclosure is to obtain an image of the skull of an object by outputting ultrasonic energy having one of resonance frequencies of a plurality of ultrasonic piezoelectric elements throughout the entire opening period of the BBB.

### [TECHNICAL SOLUTION]

According to an embodiment of the present disclosure, a focused ultrasound processing system for opening and monitoring a blood-brain barrier (BBB) may include at least one piezoelectric element consisting of a single structure and a drive module that provides an electrical signal such that ultrasonic energy having one of a plurality of resonance frequencies or pieces of ultrasonic energy having at least two resonance frequencies are output by the at least one piezoelectric element.

Moreover, according to an embodiment of the present disclosure, a member of the at least one piezoelectric element may be composed of a single material or a plurality of materials.

Furthermore, according to an embodiment of the present disclosure, the at least one piezoelectric element may output the ultrasonic energy to an object or an acoustic cavitation-inducing agent administered to the object.

Also, according to an embodiment of the present disclosure, the at least one piezoelectric element may receive an acoustic cavitation signal reflected from the object or generated due to an acoustic cavitation phenomenon of the acoustic cavitation-inducing agent.

Besides, according to an embodiment of the present disclosure, the plurality of resonance frequencies may include a first resonance frequency, and a second resonance frequency lower than the first resonance frequency.

In addition, according to an embodiment of the present disclosure, the focused ultrasound processing system for opening and monitoring the BBB may further include a determination module that generates an image of the object by analyzing the acoustic cavitation signal or determines a location or a behavior of the acoustic cavitation-inducing agent.

Moreover, according to an embodiment of the present disclosure, the determination module may generate the image by analyzing the acoustic cavitation signal generated as first ultrasonic energy having the first resonance frequency is reflected from the object.

Furthermore, according to an embodiment of the present disclosure, the determination module may generate the image by measuring Time Of Flight (TOF), which is a period between a point in time, when the first ultrasonic energy is output by the at least one piezoelectric element, and a point in time when the acoustic cavitation signal is detected.

Also, according to an embodiment of the present disclosure, the at least one piezoelectric element may open the object by outputting second ultrasonic energy having the second resonance frequency.

Besides, according to an embodiment of the present disclosure, the object may be a Blood-Brain Barrier (BBB), and the acoustic cavitation-inducing agent may be a microbubble located near the object.

**In** addition, according to an embodiment of the present disclosure, the determination module may determine the behavior of the acoustic cavitation-inducing agent by extracting a frequency component corresponding to a value of n/2 times the second resonance frequency from the acoustic cavitation signal, and the 'n' may be a natural number other than 2.

Moreover, according to an embodiment of the present disclosure, the determination module may determine the location of the acoustic cavitation-inducing agent by analyzing the acoustic cavitation signal generated by second ultrasonic energy having the second resonance frequency.

Furthermore, according to an embodiment of the present disclosure, the at least one piezoelectric element may performs at least two of
a first function of generating an image of the object by outputting the ultrasonic energy, a second function of opening the object by outputting the ultrasonic energy, a third function of determining the behavior of the acoustic cavitation-inducing agent by analyzing the acoustic cavitation signal, and a fourth function of simultaneously determining the location and the behavior of the acoustic cavitation-inducing agent by analyzing the acoustic cavitation signal.

Also, according to an embodiment of the present disclosure, a ultrasound control method of a focused ultrasound processing system for opening and monitoring BBB, which is driven by at least one processor may include providing, by the at least one processor, an electrical signal such that ultrasonic energy having at least one resonance frequency among a plurality of resonance frequencies is output, and outputting, by the at least one processor, the ultrasonic energy having the resonance frequency to an object and an acoustic cavitation-inducing agent administered to the object by receiving the electrical signal.

Besides, according to an embodiment of the present disclosure, a computer-readable recording medium recorded with a program for executing the ultrasound control method of the focused ultrasound processing system for opening and monitoring the BBB may be included.

### [ADVANTAGEOUS EFFECTS OF THE INVENTION]

According to an embodiment of the present disclosure, a focused ultrasound processing system for opening and monitoring BBB, and the control method of the focused ultrasound processing system for opening and monitoring the BBB may obtain an image of a skull by using a resonance frequency of a high-frequency region of a single-structure piezoelectric element.

Moreover, according to an embodiment of the present disclosure, a focused ultrasound processing system for opening and monitoring BBB, and the control method of the focused ultrasound processing system for opening and monitoring the BBB may open the BBB by utilizing the resonance frequency of a low-frequency region of a single-structure piezoelectric element.

Furthermore, according to an embodiment of the present disclosure, a focused ultrasound processing system for opening and monitoring BBB, and the control method of the focused ultrasound processing system for opening and monitoring the BBB may determine and monitor the focusing location (or the location of the brain where the opening of the BBB occurs) of ultrasonic energy and the opening degree of BBB (or the location and behavior of acoustic cavitation-inducing agents) by analyzing an acoustic cavitation signal generated by ultrasonic energy in real time.

### [DESCRIPTION OF THE DRAWINGS]

FIG. 1 is a diagram of a focused ultrasound processing system for opening and monitoring BBB, according to an embodiment of the present disclosure.
FIGS. 2A to 2C are diagrams of a frequency range of a single piezoelectric element, according to an embodiment of the present disclosure.
FIGS. 3A to 3C are examples of a process of obtaining an image of a skull by using a resonance frequency in a high-frequency region, according to an embodiment of the present disclosure.
FIG. 4 is another example of a process of obtaining an image of a skull by using a resonance frequency in a high-frequency region, according to an embodiment of the present disclosure.
FIG. 5 is a diagram of adjustment of a brain target location of a piezoelectric element based on image stitching, according to an embodiment of the present disclosure.
FIGS. 6A and 6B are diagrams of a process of monitoring the location and behavior of an acoustic cavitation-inducing agent and the opening of BBB by using ultrasonic energy of FIGS. 2A to 2C.
FIG. 7 is a flowchart of an ultrasound control method of a focused ultrasound processing system for opening and monitoring BBB, according to an embodiment of the present disclosure.

### [BEST MODE]

Hereinafter, various embodiments of the present disclosure will be described in detail with reference to the accompanying drawings such that those skilled in the art to which the present disclosure pertains may readily carry out the present disclosure. The present disclosure may be implemented in various different forms and is not limited to the embodiments described herein.

Components or elements not associated with the detailed description may be omitted to describe the present disclosure clearly, and the same reference numerals refer to the same or similar components throughout this application. Therefore, the reference numerals described above may be used in other drawings as well.

Moreover, the size and thickness of each of the components illustrated in the drawings are shown for convenience of explanation and the present disclosure is not necessarily limited thereto. In the drawing, the thickness may be exaggerated to clearly express various layers and regions.

Besides, an expression "the same" in the description may mean "substantially the same". In other words, it may be the same to an extent that a person with ordinary knowledge may understand that it is the same. Other expressions may also be expressions in each of which "substantially" is omitted.

Furthermore, when a portion "comprises" a component in descriptions, it will be understood that it may further include another component, without excluding other components unless specifically stated otherwise. As used herein, a "~unit" or "~part" may be a unit that processes at least one function or operation and may refer to, for example, a software, FPGA, or hardware component. The function provided by the "~unit" or "~part" may be performed separately by a plurality of components, or it may be integrated with other additional components. The "~unit" or "~part" of this specification may not be necessarily limited to software or hardware, and may be configured to be included in an addressable storage medium, or may be configured to operate one or more processors. Hereinafter, embodiments of the present disclosure will be described in detail with reference to the drawings.

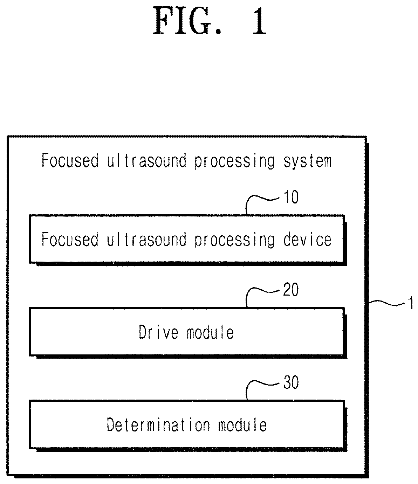

FIG. 1 is a diagram of a focused ultrasound processing system for opening and monitoring BBB, according to an embodiment of the present disclosure.

According to an embodiment of the present disclosure, a focused ultrasound processing system 1 for opening and monitoring BBB may include a focused ultrasound processing device 10, a drive module 20, and a determination module 30.

However, it is understood that the focused ultrasound processing system 1 may include fewer or more components than those illustrated in FIG. 1 to drive the focused ultrasound processing system 1 for opening and monitoring the BBB.

For example, the focused ultrasound processing system 1 for opening and monitoring the BBB may further include a power supply unit (not shown) for providing power, an output time control unit (not shown) for setting or controlling the output time of focused ultrasound energy, a resonant circuit (not shown) for setting the corresponding frequency such that focused ultrasound energy having a resonant (or center) frequency arbitrarily defined by a user is output, and a display unit (or display device) (not shown) for visually perceiving frequency analysis results.

Each of the focused ultrasound processing device 10, the drive module 20, and the determination module 30 may be implemented as at least one processor, or may be driven by the corresponding processor.

The focused ultrasound processing device 10 may be in the form of a helmet, but the present disclosure is not limited thereto. That is, the focused ultrasound processing device 10 may be manufactured in various forms capable of wrapping the head (or skull) of an object.

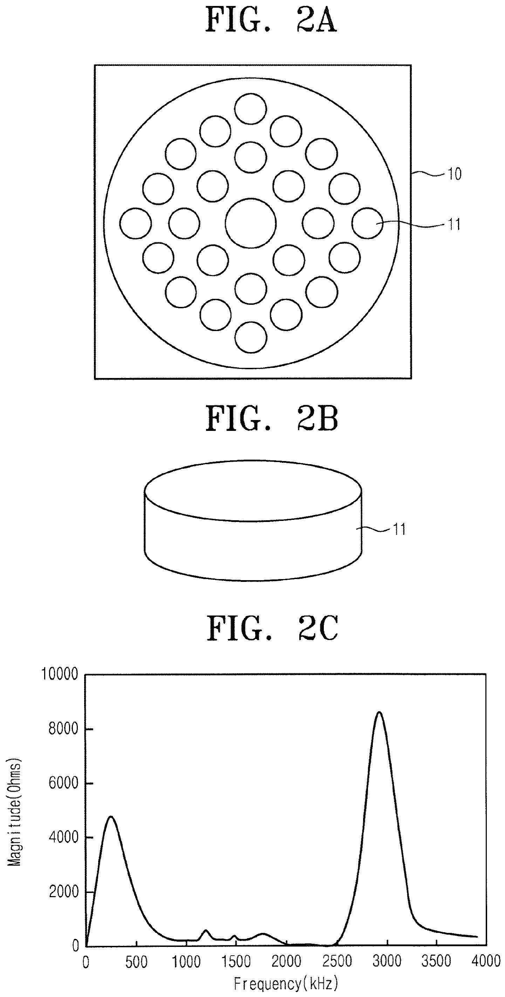

The focused ultrasound processing device 10 may include at least one piezoelectric element 11 (see FIGS. 2A and 2B). In this case, the at least one piezoelectric element 11 may be installed on the inner surface (or the lower surface) of the focused ultrasound processing device 10 facing the head (or skull) of the object.

The at least one piezoelectric element 11 may be spaced apart from another one at a predetermined distance and may be arranged on the lower surface of the focused ultrasound processing device 10. In this case, the arrangement structure of the piezoelectric element 11 may be varied. For example, at least one piezoelectric element may be arranged radially around the center of the focused ultrasound processing device 10.

Each of the at least one piezoelectric element 11 may be implemented in a single structure (see FIG. 2B). That is, the piezoelectric element 11 may be a single element formed with only one structure, rather than being composed of different separate elements that are combined.

The piezoelectric element 11 may be made of quartz, ceramic materials (e.g., lead zirconate titanate (PZT)), polymer materials, etc., but the present disclosure is not limited thereto.

The member of the piezoelectric element 11 interacting with ultrasonic energy (or focused ultrasound energy) described later may be composed of at least one of the materials described above. For example, the piezoelectric element 11 may be made of one of the materials described above or a mixture of at least two of the materials described above.

Each of the at least one piezoelectric element 11 may output ultrasonic energy to at least one of the object and an acoustic cavitation-inducing agent based on an electrical signal provided from the drive module 20 described below.

In detail, as described below, the acoustic cavitation-inducing agent may be located in the vicinity of the object, and thus the ultrasonic energy output from the at least one piezoelectric element 11 may reach not only the object (e.g., BBB) but also the acoustic cavitation-inducing agent.

In addition, the at least one piezoelectric elements 11 may receive (or detect) an acoustic cavitation signal (hereinafter, referred to as an "acoustic signal") generated by the acoustic cavitation phenomenon of the acoustic cavitation-inducing agent.

In particular, the acoustic cavitation-inducing agent (e.g., a microbubble) may be pre-injected into the object. The microbubble, which is the acoustic cavitation-inducing agent, may be located near the BBB after being injected into the object.

The at least one piezoelectric element 11 may output ultrasonic energy to at least one of the target (e.g., BBB) of the object and the pre-injected microbubble based on an electrical signal.

The microbubble receiving the ultrasonic energy may generate the acoustic signal due to the acoustic cavitation phenomenon, and the at least one piezoelectric element 11 may receive (or detect) the corresponding acoustic signal.

The drive module 20 may provide an electrical signal such that ultrasonic energy having at least one resonance frequency among a plurality of resonance frequencies is simultaneously or sequentially output by the at least one piezoelectric element 11.

In this case, the plurality of resonance frequencies may include a first resonance frequency (or a resonance frequency in a high-frequency range) and a second resonance frequency (or a resonance frequency in a low-frequency range) lower than the first resonance frequency. In this case, the frequency values of the first resonance frequency and the second resonance frequency may be set in various ways in advance by the user.

The drive module 20 may provide the electrical signal to the corresponding piezoelectric element 11 such that the at least one piezoelectric element 11 simultaneously or sequentially outputs ultrasonic energy having at least one resonance frequency among the first resonance frequency and the second resonance frequency according to the user's settings or preset conditions.

For example, the drive module 20 may provide the electrical signal (hereinafter referred to as a "first electrical signal") to the arbitrary pre-specified piezoelectric element 11, and the corresponding piezoelectric element 11 may output ultrasonic energy (hereinafter referred to as "first ultrasonic energy") having the first resonance frequency based on the first electrical signal to at least one of the object and the acoustic cavitation-inducing agent.

Alternatively, the drive module 20 may provide the electrical signal (hereinafter referred to as a "second electrical signal") to the arbitrary piezoelectric elements 11, and the corresponding piezoelectric element 11 may output ultrasonic energy (hereinafter referred to as a "second ultrasonic energy") having the second resonance frequency based on the second electrical signal to at least one of the object (or BBB) and the acoustic cavitation-inducing agent.

Alternatively, the drive module 20 may provide the electrical signal to the arbitrary piezoelectric element 11 or the plurality of piezoelectric elements 11, and the corresponding piezoelectric element 11 may simultaneously or sequentially outputs the first ultrasonic energy having the first resonance frequency and the second ultrasonic energy having the second resonance frequency to at least one of the object and the acoustic cavitation-inducing agent, based on the electrical signal.

In this case, the object (or BBB) receiving the second ultrasonic energy may be opened. The opening of the BBB allows the desired drug to enter the brain.

The determination module 30 may analyze the acoustic signal to generate an image of the object or to determine the focusing location (or the location of the brain where the opening of the BBB occurs, the current location or behavior of the acoustic cavitation-inducing agent, or the opening degree of the BBB) of ultrasonic energy.

In particular, the determination module 30 may generate an image (e.g., an image of the object's skull) by analyzing the acoustic signal generated when the first ultrasonic energy having the first resonance frequency is reflected from the object.

In this case, the determination module 30 may generate an image by measuring Time Of Flight (TOF), which is a period between a point in time, when the first ultrasonic energy is output by the at least one piezoelectric element 11, and a point in time when it is reflected back and detected.

Alternatively, the determination module 30 may determine the focusing location (or the location of the brain where the opening of the BBB occurs, the current location or behavior of the acoustic cavitation-inducing agent, or the opening degree of the BBB) of ultrasonic energy by analyzing the acoustic signal generated when the second ultrasonic energy having the second resonance frequency is reflected from the object.

In detail, the determination module 30 may determine the behavior of the acoustic cavitation-inducing agent by extracting the frequency component (i.e., a sub-harmonics component, a harmonics component, and an ultra-harmonics component) corresponding to n/2 times (where 'n' is a natural number other than 2) the second resonance frequency from the acoustic signal.

The behavior of the acoustic cavitation-inducing agent refers to the size, shape, etc. of the microbubble that vibrates due to ultrasonic energy, and the opening degree of BBB may be inferred (or determined) from the behavior of the acoustic cavitation-inducing agent.

Alternatively, the determination module 30 may determine the location (or the current location) of the acoustic cavitation-inducing agent by analyzing (or Passive Acoustic Mapping (PAM)) a plurality of acoustic signals detected by the plurality of piezoelectric elements 11.

In the meantime, the at least one piezoelectric element 11 described above may simultaneously or sequentially perform at least two of a first function for generating an image of an object by outputting ultrasonic energy, a second function for opening the BBB by outputting ultrasonic energy, a third function for determining the behavior of an acoustic cavitation-inducing agent by analyzing an acoustic signal, and a fourth function for simultaneously determining the location and behavior of an acoustic cavitation-inducing agent by analyzing the acoustic signal.

For example, the arbitrary piezoelectric element 11 may simultaneously or sequentially perform the first function that outputs ultrasonic energy to the skull of an object to obtain an image of the skull, and the third function that determines the behavior of the acoustic cavitation-inducing agent by analyzing the acoustic signal received from the acoustic cavitation-inducing agent.

Alternatively, the arbitrary piezoelectric element 11 may simultaneously or sequentially perform the second function that outputs ultrasonic energy to the BBB to open the BBB, and the fourth function that simultaneously determines the location and behavior of the acoustic cavitation-inducing agent by analyzing the acoustic signal received from the acoustic cavitation-inducing agent.

FIGS. 2A to 2C are diagrams of a frequency range of a single piezoelectric element, according to an embodiment of the present disclosure.

FIG. 2A is a view of the bottom surface of the focused ultrasound processing device 10. FIG. 2B is a perspective view of a piezoelectric element. FIG. 2C is a graph of the frequency range utilized by the at least one piezoelectric element 11 provided in the connected ultrasonic processing device 10.

Referring to FIG. 2A, a through-opening may be formed at the center of the focused ultrasound processing device 10. In this case, the at least one piezoelectric element 11 may be spaced apart from another at a predetermined interval around the through-opening and may be arranged radially.

As described above in FIG. 1, the member of the piezoelectric element 11 which interacts with ultrasonic energy may be composed of a single material or a plurality of materials (e.g., a composite material with different densities).

In FIG. 2B, the piezoelectric element 11 is illustrated in the form of a cylinder, but the present disclosure is not limited thereto. That is, the piezoelectric element 11 may be formed into various three-dimensional shapes capable interacting with ultrasonic energy.

Moreover, each of the plurality of piezoelectric elements 11 may perform at least two of the first to fourth functions described above in FIG. 1. This may be preset by a user.

The drive module 20 may perform the first function and may provide a first electrical signal to the predetermined (or specified) piezoelectric element 11. In this case, the corresponding piezoelectric element 11 may output, to an object, the first ultrasonic energy having the first resonance frequency (approximately 3000 kHz) in the frequency range shown in FIG. 2C.

Alternatively, the drive module 20 may perform the second function and may provide a second electrical signal to the predetermined (or specified) piezoelectric element 11. In this case, the piezoelectric element 11 may output second ultrasonic energy having a second resonance frequency (about 250 kHz) in the frequency range shown in FIG. 2C to at least one of the object (or BBB) and the acoustic cavitation-inducing agent.

In the meantime, the piezoelectric element 11 configured to perform the third function may receive an acoustic signal generated by the acoustic cavitation-inducing agent and may provide the acoustic signal to the determination module 30. The determination module 30 may determine the behavior of the acoustic cavitation-inducing agent by analyzing a frequency component (125 kHz, 375 kHz, ...) corresponding to the value of n/2 times (i.e., f2*n/2, n = 1, 3, 5, ...) the second resonance frequency (250 kHz, f2), and a frequency component (500 kHz, 750 kHz, ...) corresponding to the value of f2*n (n = 2, 3, 4, ...) in the acoustic signal.

The piezoelectric element 11 configured to perform the fourth function may receive a plurality of acoustic signals generated by the acoustic cavitation-inducing agent and may provide the plurality of acoustic signals to the determination module 30. The determination module 30 may perform PAM based on the plurality of acoustic signals and may simultaneously determine the location and behavior of the acoustic cavitation-inducing agent.

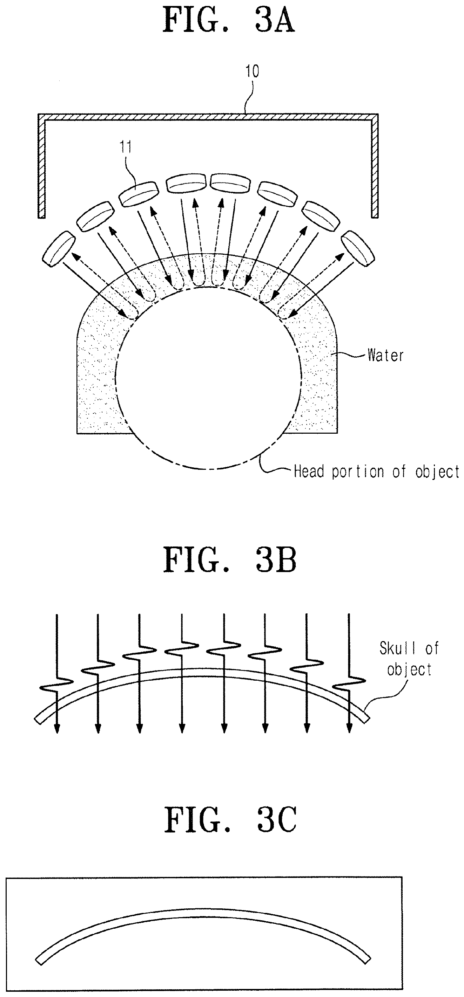

FIGS. 3A to 3C are examples of a process of obtaining an image of a skull by using a resonance frequency in a high-frequency region, according to an embodiment of the present disclosure.

Referring to FIG. 3A, the focused ultrasound processing device 10 according to an embodiment of the present disclosure may be mounted on the head portion of an object. In this case, a medium that facilitates the transmission of focused ultrasound energy may be positioned between the at least one piezoelectric element 11 and the head portion of the object.

For example, the medium may be water, but the present disclosure is not limited thereto. That is, a material capable of minimizing the attenuation of ultrasonic energy may be used instead of water.

Referring to FIGS. 1, 3A, and 3B together, to obtain a skull image of the object, the drive module 20 may provide a first electrical signal to the at least one piezoelectric element 11 that performs a first function.

The piezoelectric element 11 may output the first ultrasonic energy (solid line in FIGS. 3A and 3B) having the first resonance frequency (about 3000 kHz) to the skull of the object based on the first electrical signal.

The first ultrasonic energy may be output from the piezoelectric element 11 and then reach the surface of the object's skull, and may be reflected from the surface of the skull (as shown in FIG. 3A) and return to the piezoelectric element 11.

In this case, the determination module 30 may generate an image of the skull (or a brightness-mode (B-mode) image, see FIG. 3C) by measuring TOF, which is a period between a point in time, when the first ultrasonic energy is output from the corresponding piezoelectric element 11, and a point in time when the first ultrasonic energy reflected from the surface of the skull is detected.

The determination module 30 may generate the image of the skull through the process, and a user may visually perceive the image through a display unit (or a display device).

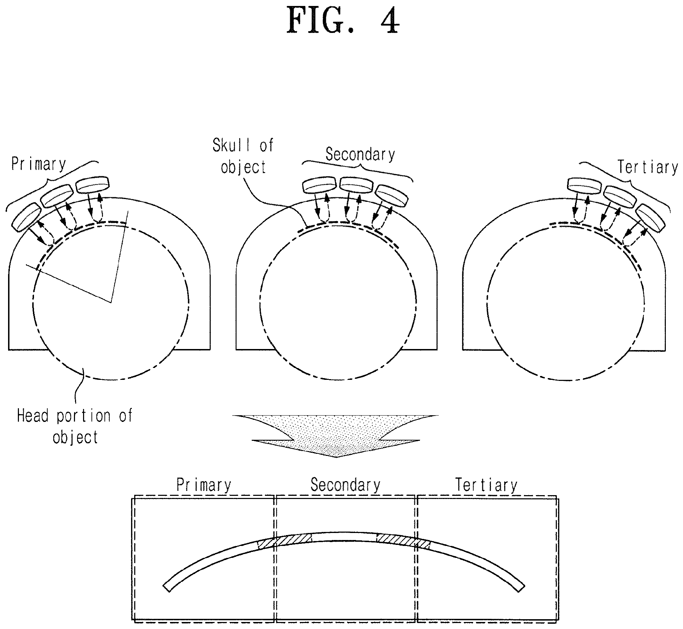

FIG. 4 is another example of a process of obtaining an image of a skull by using a resonance frequency in a high-frequency region, according to an embodiment of the present disclosure.

Referring to FIG. 4, unlike the FIGS. 3A to 3C, a skull image of an object may be obtained while the location of the at least one piezoelectric element 11 provided in the focused ultrasound processing device 10 is moved.

Specifically, when it is difficult to obtain a full image of the object's skull by using only the at least one piezoelectric element 11 of the focused ultrasound processing device 10 due to limitations such as the area of the focused ultrasound processing device 10, segmented images of the skull may be obtained and then synthesized (or overlapped) to obtain the full image of the skull.

For example, the at least one piezoelectric element 11 installed at the first location of the focused ultrasound processing device 10 may output (primary output in FIG. 4) the first ultrasonic energy having the first resonance frequency to the first region of the skull.

The corresponding piezoelectric element 11 may receive the first ultrasonic energy reflected and returned from the surface of the first region of the skull, and the determination module 30 may generate an image of the first region of the skull by using the TOF described above in FIGS. 3A to 3C.

The at least one piezoelectric element 11 may be moved to the second location of the focused ultrasound processing device 10 by a moving device installed in the focused ultrasound processing device 10 or by a user's manipulation.

The moved at least one piezoelectric element 11 may output (secondary output in FIG. 4) the first ultrasonic energy having the first resonance frequency to the second region of the skull.

The corresponding piezoelectric element 11 may receive the first ultrasonic energy reflected and returned from the surface of the second region of the skull, and the determination module 30 may generate an image of the second region of the skull by using the TOF described above in FIGS. 3A to 3C.

The at least one piezoelectric element 11 may be moved to the third location of the focused ultrasound processing device 10 by the moving device installed in the focused ultrasound processing device 10 or by the user's manipulation.

The moved at least one piezoelectric element 11 may output (tertiary output in FIG. 4) the first ultrasonic energy having the first resonance frequency to the third region of the skull.

The corresponding piezoelectric element 11 may receive the first ultrasonic energy reflected and returned from the surface of the third region of the skull, and the determination module 30 may generate an image of the third region of the skull by using the TOF described above in FIGS. 3A to 3C.

The determination module 30 may obtain an image of the entire skull overlapped by synthesizing images of the first to third regions of the skull generated through the above-described process.

Meanwhile, in FIG. 4, a two-dimensional image of a skull is shown, but the present disclosure is not limited thereto. That is, the shape of the skull may be three-dimensional. Through the above-described process, the determination module 30 may generate a three-dimensional image of the entire skull.

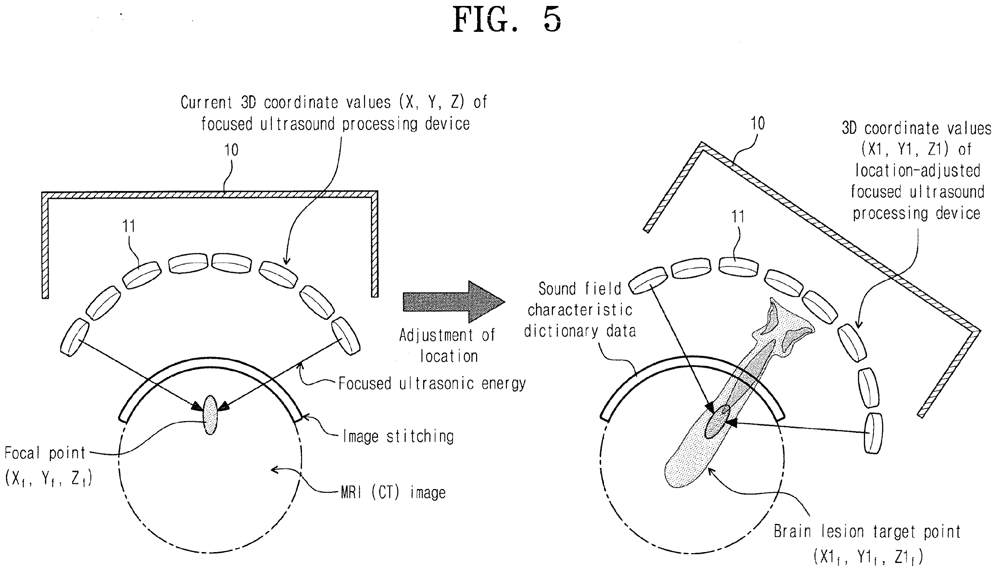

FIG. 5 is a diagram of adjustment of a brain target location of a piezoelectric element based on image stitching, according to an embodiment of the present disclosure.

An MRI image (or a CT image) of an object may be stored in a database (not shown). That is, the MRI image (or the CT image) of the object may be obtained on-site by using an MRI device (or a CT device, not shown), and the corresponding image may be stored in the database.

The determination module 30 may move the focused ultrasound processing device 10 by using the image of the skull portion or the entire skull described in FIGS. 3 and 4, and the MRI image (or CT image) pre-stored in the database, thereby adjusting a brain target location where the ultrasonic energy is focused.

In detail, the determination module 30 may compare and analyze an image of all or part of the skull with the pre-stored MRI image (or the CT image) through an image stitching process.

For example, the determination module 30 may obtain the current 3D coordinate values (X, Y, Z) of the focused ultrasound processing device 10 in the case where the skull is maximally aligned through comparative analysis based on the image stitching process (or a point where an error rate (or an image stitching error rate) between images is the lowest), and the focal point coordinate values (Xf, Yf, Zf) of the ultrasonic energy output from the focused ultrasound processing device 10 located at the corresponding 3D coordinate values (X, Y, Z).

A moving device (e.g., a robotic arm) may adjust a location of the focused ultrasound processing device 10 based on the obtained current 3D coordinate values (X, Y, Z) of the focused ultrasound processing device 10 to move the location of the focused ultrasound processing device 10 to a location of new 3D coordinate values (X1, Y1, Z1).

The moving device may consider brain lesion target location coordinates (X1f, Y1f, Z1f) obtained from the MRI (or CT) image, and may adjust the location of the focused ultrasound processing device 10 from the current 3D coordinate values (X, Y, Z) to new 3D coordinate values (X1, Y1, Z1) such that the coordinate values (Xf, Yf, Zf) of the current focal point of the ultrasound energy are positioned at the coordinate values (X1f, Y1f, Z1f) of the corresponding brain lesion target location.

In this case, the sound field characteristic dictionary data of the focused ultrasound processing device 10 may be used. In detail, the sound field characteristic dictionary data refers to data regarding the different focusing characteristics of the ultrasonic energy output depending on the location (e.g., an installation location, an installation angle, etc.) of the focused ultrasound processing device 10 based on the object's skull, such as the location of a focal point where ultrasonic energy output from the at least one piezoelectric element 11 included in the focused ultrasound processing device 10 is fused and focused, the width of the focal point, and the length of the focal point.

The sound field characteristic dictionary data for each location of the focused ultrasound processing device 10 based on the object's skull may be pre-stored in the database. The moving device (e.g., a robotic arm) may move the focused ultrasound processing device 10 or may adjust the location of at least one piezoelectric element 11 such that the ultrasound energy may be effectively focused on the target focusing location (X1f, Y1f, Z1f) by using the sound field characteristic dictionary data.

In particular, the moving device may move the focused ultrasound processing device 10 or may adjust (e.g., forward or backward placements of the piezoelectric element 11) the location of at least one piezoelectric element 11 such that the ultrasound energy may be focused on a target focusing location (or a target focal point) determined through clinical analysis (or MRI imaging) by using the sound field characteristic dictionary data.

The process of moving the focused ultrasound processing device 10 or adjusting the location of the at least one piezoelectric element 11 may include a process of moving or rotating the at least one piezoelectric element 11 by using the moving device.

Alternatively, the process may include a process of increasing or decreasing the distance of the focused ultrasound processing device 10 based on the surface of the skull by using the moving device.

Alternatively, the process may include a process of moving the at least one piezoelectric element 11 such that the at least one piezoelectric element 11 included in the focused ultrasound processing device 10 may be moved closer to or further away from the surface of the skull by using the moving device.

Through the above-described process, the ultrasonic energy may be focused on the brain lesion target. The determination module 30 may obtain a 3D target focal point (or brain lesion target point (X1f, Y1f, Z1f)) and new 3D coordinate values (X1, Y1, Z1) of the adjusted focused ultrasound processing device 10 through the above-described process.

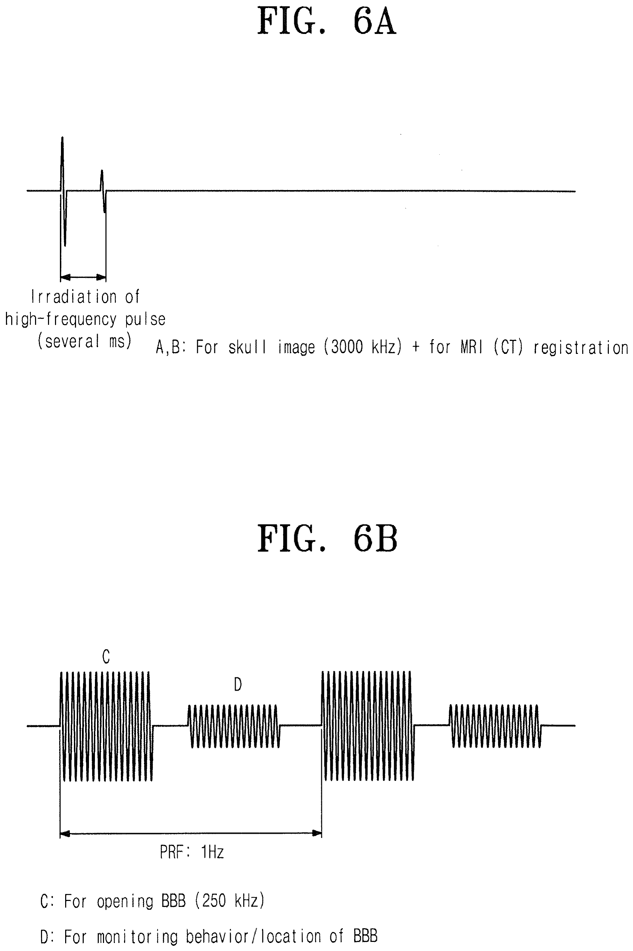

FIGS. 6A and 6B are diagrams of a process of monitoring the location and behavior of an acoustic cavitation-inducing agent and the opening of BBB by using ultrasonic energy of FIGS. 2A to 2C.

Referring to FIGS. 2 and 6A together, the at least one piezoelectric element 11 performing a first function based on a first electrical signal provided from the drive module 20 may output a pulse 'A' of ultrasonic energy of a first resonance frequency (3000 kHz) of a high-frequency region.

The at least one piezoelectric element 11 may detect a pulse 'B' of ultrasonic energy that is reflected and return from an object's skull. In this case, a time interval between a point in time when the pulse 'A' is output from the at least one piezoelectric element 11 and a point in time when the pulse 'B' is detected by the piezoelectric element 11 may be several milliseconds (ms).

The determination module 30 may generate an image of the skull by measuring TOF, which is the time interval (several ms) between the point in time when the pulse 'A' is output from the at least one piezoelectric element 11 and the point in time when the pulse 'B' is detected by the piezoelectric element 11.

In the meantime, it is described that an image of an object's skull is generated by using the pulses 'A' and 'B', but the present disclosure is not limited thereto. That is, the pulses 'A' and 'B' may also be used in the image stitching process with the MRI image (or CT image) described above in FIG. 5.

Referring to FIGS. 2 and 6B together, the at least one piezoelectric element 11 performing a second function based on a second electrical signal provided from the drive module 20 may output a pulse 'C' of ultrasonic energy of a second resonance frequency (250 kHz) of a low-frequency region.

The pulse 'C' of ultrasonic energy may be output to the BBB of the desired brain tissue of the object, and the BBB may be opened due to the pulse 'C' of ultrasonic energy.

In this case, because an acoustic cavitation-inducing agent (a microbubble) may be located not only at the BBB but also near the BBB, the output ultrasonic energy may be applied (or may reach) not only to the BBB but also to the acoustic cavitation-inducing agent.

The acoustic cavitation-inducing agent may cause a resonance phenomenon due to the pulse 'C' of ultrasonic energy and may emit the acoustic signal 'D' to the outside due to the acoustic cavity phenomenon.

The piezoelectric element 11, which performs a third function, may detect the acoustic signal 'D' emitted from the acoustic cavitation-inducing agent, and the determination module 30 may analyze the acoustic signal 'D' in real time.

The determination module 30 may determine the behavior of the acoustic cavitation-inducing agent by analyzing a frequency component (125 kHz, 375 kHz, ...) corresponding to the value of n/2 times (i.e., f2*n/2, n = 1, 3, 5, ...) the second resonance frequency (250 kHz, f2), and a frequency component (500 kHz, 750 kHz, ...) corresponding to the value of 'n' times (f2*n, n = 2, 3, 4, ...) the second resonance frequency (250 kHz, f2) in the acoustic signal 'D'.

That is, as described above in FIGS. 6A and 6B, the present disclosure may simultaneously output ultrasonic energy to the BBB and an acoustic cavitation-inducing agent, while analyzing the acoustic signal generated from the acoustic cavitation-inducing agent to analyze the behavior (e.g., the intensity of behavior) of the acoustic cavitation-inducing agent, thereby determining whether the BBB is opened appropriately and safely.

In addition, the determination module 30 may perform PAM by analyzing the plurality of acoustic signals 'D' detected by the at least one piezoelectric element 11 (or the plurality of piezoelectric elements 11) performing a fourth function, and may simultaneously determine (or monitor) the location and behavior of the acoustic cavitation-inducing agent.

The process may be repeated during a Pulse Repetition Frequency (PRF) of 1 Hz, but the present disclosure is not limited thereto. That is, the above-described process may be repeated during various periods of time set by the user.

As described above, the focused ultrasound processing system 1 for opening and monitoring BBB of the present disclosure may open the BBB by utilizing low-frequency-based ultrasound energy with optimized skull penetration efficiency, may obtain images of the skull by utilizing high-frequency-based ultrasound energy in the ultrasound energy output process, and may adjust the location of the focused ultrasound processing device 10 or control the piezoelectric element 11 in real time through image stitching, thereby irradiating the ultrasonic energy to a desired lesion.

That is, the present disclosure may obtain images of the skull by using ultrasonic energy with a high resonance frequency, may adjust the location (or an installation location, an installation angle, etc.) of the focused ultrasound processing device 10 so as to suit the irradiation of the ultrasonic energy, and may open the BBB by using ultrasonic energy with a low resonance frequency, thereby easily and safely moving to the brain.

Moreover, the opening degree of BBB in real time may be inferred by analyzing the acoustic signal generated by an acoustic cavitation-inducing agent located near the BBB in real time, and whether the ultrasonic energy is being properly focused may be determined or monitored by identifying the current location of the acoustic cavitation-inducing agent.

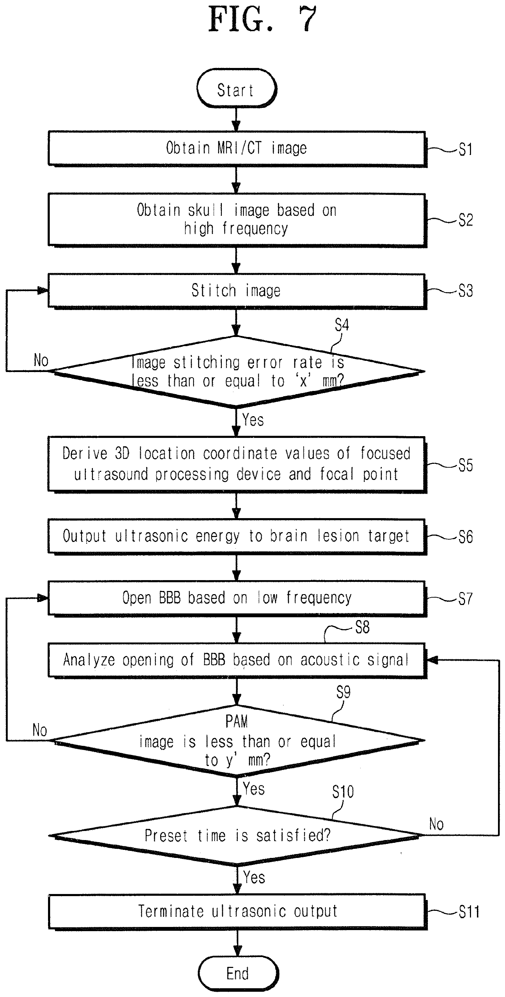

FIG. 7 is a flowchart of an ultrasound control method of a focused ultrasound processing system for opening and monitoring BBB, according to an embodiment of the present disclosure.

In step S1, an MRI image or a CT image may be obtained.

In detail, the determination module 30 may upload the MRI image or the CT image of an object pre-stored in a database.

In step S2, a skull image may be obtained based on a high frequency.

In detail, the focused ultrasound processing device 10 may be mounted in advance on the head portion of the object. The drive module 20 may perform a first function and may provide a first electrical signal to the predetermined at least one piezoelectric element 11.

The focused ultrasound processing device 10 may output the first ultrasonic energy having the first resonance frequency (about 3000 kHz) to the skull of the object based on the first electrical signal.

The first ultrasonic energy may be output from the piezoelectric element 11 and then reach the surface of the object's skull, and may be reflected from the surface of the skull and return to the piezoelectric element 11.

In this case, the determination module 30 may obtain an image of the skull by measuring TOF, which is a period between a point in time, when the first ultrasonic energy is output from the corresponding piezoelectric element 11, and a point in time when the first ultrasonic energy reflected from the surface of the skull is detected.

In step S3, the images may be stitched.

In detail, the determination module 30 may perform image stitching by comparing and analyzing the MRI image or CT image of the object obtained in step S1 and the image of the skull obtained in step S2.

In step S4, it may be determined whether an image stitching error rate is "x' mm or less.

In detail, the determination module 30 may determine whether an image stitching error rate between the MRI image or CT image of the object obtained in step S1 and the image of the skull obtained in step S2 is less than or equal to a preset 'x' mm.

When the image stitching error rate exceeds the preset 'x' mm, the process returns to step S3, and the skull image obtained in step S2 may be adjusted (e.g., rotated) and image stitching may be performed again with the MRI image or CT image of the object obtained in step S1. This may be repeated until the image stitching error rate is less than the preset 'x' mm.

In step S5, the current location coordinate value of the focused ultrasound processing device and the current location coordinate value of the focal point may be derived.

Specifically, when the image stitching error rate is less than a preset value of 'x' mm (i.e., corresponding to a point where the error rate between images is the lowest, or the case where image stitching is complete), the determination module 30 may obtain the current 3D coordinate values (X, Y, Z) of the focused ultrasound processing device 10 mounted on the object.

Furthermore, as described above in FIG. 5, the determination module 30 may obtain the focal point coordinate values (Xf, Yf, Zf) of the ultrasonic energy output at the current location based on the obtained current 3D coordinate values (X, Y, Z) of the focused ultrasound processing device 10 (i.e., by utilizing sound field characteristic dictionary data).

As described above in FIG. 5, a moving device may move the focused ultrasound processing device 10 by additionally utilizing the sound field characteristic dictionary data or may adjust the location of the at least one piezoelectric element 11 to obtain new 3D coordinate values (X1, Y1, Z1) of the adjusted focused ultrasound processing device 10 and new 3D target focal point coordinate values (Xf1, Yf1, Zf1) of the ultrasonic energy.

In step S6, the ultrasonic energy may be output to a brain lesion target.

That is, at the current three-dimensional coordinate values (X1, Y1, Z1) of the focused ultrasound processing device 10 obtained in step S5, the at least one piezoelectric element 11 may output the second ultrasonic energy having a low second resonance frequency (about 250 kHz) to the object (e.g., a brain lesion target).

In detail, the drive module 20 may perform a second function and may provide a second electrical signal to the predetermined piezoelectric element 11. In this case, the focused ultrasound processing device 10 may output second ultrasonic energy having a second resonance frequency (approximately 250 kHz) to the BBB and a microbubble located near the BBB.

In step S7, the BBB may be opened.

In detail, the second ultrasonic energy output from the at least one piezoelectric element 11 may reach the BBB and the microbubble located nearby, and thus the BBB may be opened.

In step S8, the opening of the BBB may be analyzed based on an acoustic signal.

In particular, the piezoelectric element 11 configured to perform a third function may receive an acoustic signal generated by an acoustic cavitation-inducing agent. The acoustic signal may be provided to the determination module 30.

The determination module 30 may determine the behavior of the acoustic cavitation-inducing agent by analyzing a frequency component (125 kHz, 375 kHz, ...) corresponding to the value of n/2 times (i.e., f2*n/2, n = 1, 3, 5, ...) the second resonance frequency (250 kHz, f2), and a frequency component (500 kHz, 750 kHz, ...) corresponding to the value of f2*n (n = 2, 3, 4, ...) in the acoustic signal.

When the determination result of the behavior of an acoustic cavitation-inducing agent indicates that the surrounding tissue cells are damaged beyond the opening of the BBB, for example, the case where the microbubble is about to break, the intensity of the ultrasonic energy may be reduced.

Alternatively, the piezoelectric element 11 configured to perform the fourth function may receive a plurality of acoustic signals generated by the acoustic cavitation-inducing agent and may provide the plurality of acoustic signals to the determination module 30. The determination module 30 may perform PAM based on the plurality of acoustic signals and may simultaneously determine the location and behavior of the acoustic cavitation-inducing agent.

In this way, it is possible to monitor whether the ultrasonic energy is focused at the desired location as a result of the location determination of the acoustic cavitation-inducing agent. When it is not focused at the desired location, the location of the piezoelectric element may be adjusted as in step S6.

In step S9, it is possible to determine whether the error rate is less than or equal to 'y' mm, by using an image based on PAM.

In detail, the determination module 30 may obtain a PAM image based on the PAM performed in step S8. Furthermore, the determination module 30 may determine whether the error rate of the PAM image is less than or equal to 'y' mm.

When the error rate of the PAM image exceeds 'y' mm, the focal point location of the ultrasound energy output at the current location of the focused ultrasound processing device 10 does not properly match the location of the brain lesion target, and thus the procedure may return to step S6, may reset the focal point coordinate value of the ultrasound energy output from the focused ultrasound processing device 10, and may output the ultrasound energy again.

Steps S6 to S9 may be repeated until the error rate of the PAM image is less than or equal to 'y' mm, and may reset the focal point of ultrasonic energy in real time during a process of opening the BBB by using ultrasonic energy. In this way, the opening accuracy of the BBB may be improved.

The pre-stored sound field characteristic dictionary data may be utilized in a method for resetting the focal point of ultrasonic energy, but the present disclosure is not limited thereto.

In step S10, whether a preset time is satisfied may be determined.

In detail, when the error rate of the PAM image in step S9 is less than or equal to 'y' mm, the focal point location of the ultrasound energy output at the current location of the focused ultrasound processing device 10 correctly matches the location of the brain lesion target, and thus the determination module 30 may determine whether the preset time (e.g., treatment time) is satisfied. The preset time may be variously set by a user.

When the preset time is not satisfied, the procedure may return to step S7 and may open the BBB again based on the low frequency. That is, steps S7 to S10 may be repeated until the preset time is satisfied.

In step S11, the output of ultrasonic energy may be terminated.

In particular, the output of ultrasonic energy may be terminated when the preset time (e.g., a treatment time) is satisfied.

The drawings and detailed description of the present disclosure given so far are merely illustrative of the present disclosure. This is only used for the purpose of describing the present disclosure and is not used to limit the scope of the present disclosure described in the meaning or claims. Therefore, it will be understood that various modifications and other equivalent embodiments are possible from this point by those skilled in the art. The technical protection scope of the present disclosure will be defined by the technical spirit of the appended claims.

The above-described embodiments may be implemented with hardware components, software components, and/or a combination of hardware components and software components. For example, the devices, methods, and elements described in the embodiments of the present disclosure may be implemented by using one or more general-use computers or special-purpose computers, such as a processor, a controller, an arithmetic logic unit (ALU), a digital signal processor, a microcomputer, a field programmable gate array (FPGA), a programmable logic unit (PLU), a microprocessor, or any device which may execute instructions and respond.

A processing unit may perform an operating system (OS) or one or software applications running on the OS. Further, the processing device may access, store, manipulate, process and generate data in response to execution of software. It will be understood by those skilled in the art that although a single processing unit may be illustrated for convenience of understanding, the processing unit may include a plurality of processing elements and/or a plurality of types of processing elements.

For example, the processing unit may include a plurality of processors or one processor and one controller. Also, the processing unit may include a different processing configuration, such as a parallel processor. Software may include computer programs, codes, instructions or one or more combinations thereof and configure a processing unit to operate in a desired manner or independently or collectively control the processing unit.

Software and/or data may be embodied in any type of machine, components, physical equipment, virtual equipment, computer storage media or devices so as to be interpreted by the processing unit or to provide instructions or data to the processing unit. Software may be dispersed throughout computer systems connected via networks and be stored or executed in a dispersion manner. Software and data may be recorded in one or more computer-readable storage media.

The methods according to the above-described embodiments may be recorded in a computer-readable medium including program instructions that are executable via various computer devices. The non-transitory computer-readable medium may include program instructions, data files, data structures, etc. independently or may include a combination thereof. The program instructions recorded in the media may be designed and configured specially for the exemplary embodiments of the present disclosure or be known and available to those skilled in computer software.

The computer-readable medium may include a hardware device, which is specially configured to store and execute program instructions, such as magnetic media (e.g., a hard disk drive, a floppy disk, and a magnetic tape), optical media (e.g., CD-ROM and DVD), read only memories (ROMs), random access memories (RAMs), and flash memories. Examples of computer programs include not only machine language codes created by a compiler, but also high-level language codes that are capable of being executed by a computer by using an interpreter or the like. The described hardware devices may be configured to act as one or more software modules to perform the operations of the above-described embodiments, or vice versa.

While embodiments have been shown and described with reference to the accompanying drawings, it will be apparent to those skilled in the art that various modifications and variations may be made from the foregoing descriptions. For example, adequate effects may be achieved even though the foregoing processes and methods are carried out in different order than described above, and/or the aforementioned elements, such as systems, structures, devices, or circuits, are combined or coupled in different forms and modes than as described above or be substituted or switched with other components or equivalents. Therefore, other implements, other embodiments, and equivalents to claims are within the scope of the following claims.

[INDUSTRIAL APPLICABILITY]

## Claims

1. A focused ultrasound processing system for opening and monitoring a blood-brain barrier (BBB), the system comprising:
at least one piezoelectric element consisting of a single structure; and
a drive module configured to provide an electrical signal such that ultrasonic energy having one of a plurality of resonance frequencies or pieces of ultrasonic energy having at least two resonance frequencies are output by the at least one piezoelectric element.

2. The system of claim 1, wherein a member of the at least one piezoelectric element is composed of a single material or a plurality of materials.

3. The system of claim 1, wherein the at least one piezoelectric element outputs the ultrasonic energy to an object or an acoustic cavitation-inducing agent administered to the object.

4. The system of claim 3, wherein the at least one piezoelectric element receives an acoustic cavitation signal reflected from the object or generated due to an acoustic cavitation phenomenon of the acoustic cavitation-inducing agent.

5. The system of claim 4, wherein the plurality of resonance frequencies includes:
a first resonance frequency; and
a second resonance frequency lower than the first resonance frequency.

6. The system of claim 5, further comprising:
a determination module configured to generate an image of the object by analyzing the acoustic cavitation signal or to determine a location or a behavior of the acoustic cavitation-inducing agent.

7. The system of claim 6, wherein the determination module generates the image by analyzing the acoustic cavitation signal generated as first ultrasonic energy having the first resonance frequency is reflected from the object.

8. The system of claim 7, wherein the determination module generates the image by measuring Time Of Flight (TOF), which is a period between a point in time, when the first ultrasonic energy is output by the at least one piezoelectric element, and a point in time when the acoustic cavitation signal is detected.

9. The system of claim 5, wherein the at least one piezoelectric element opens the object by outputting second ultrasonic energy having the second resonance frequency.

10. The system of claim 9, wherein the object is a Blood-Brain Barrier (BBB), and the acoustic cavitation-inducing agent is a microbubble located near the object.

11. The system of claim 6, wherein the determination module determines the behavior of the acoustic cavitation-inducing agent by extracting a frequency component corresponding to a value of n/2 times the second resonance frequency from the acoustic cavitation signal, and
wherein the 'n' is a natural number other than 2.

12. The system of claim 6, wherein the determination module determines the location of the acoustic cavitation-inducing agent by analyzing the acoustic cavitation signal generated by second ultrasonic energy having the second resonance frequency.

13. The system of claim 6, wherein the at least one piezoelectric element performs at least two of:
a first function of generating an image of the object by outputting the ultrasonic energy;
a second function of opening the object by outputting the ultrasonic energy;
a third function of determining the behavior of the acoustic cavitation-inducing agent by analyzing the acoustic cavitation signal; and
a fourth function of simultaneously determining the location and the behavior of the acoustic cavitation-inducing agent by analyzing the acoustic cavitation signal.

14. An ultrasound control method of a focused ultrasound processing system for opening and monitoring BBB, which is driven by at least one processor, the method comprising:
providing, by the at least one processor, an electrical signal such that ultrasonic energy having at least one resonance frequency among a plurality of resonance frequencies is output; and
outputting, by the at least one processor, the ultrasonic energy having the resonance frequency to an object and an acoustic cavitation-inducing agent administered to the object by receiving the electrical signal.

15. A computer-readable recording medium recorded with a program for executing the ultrasound control method of the focused ultrasound processing system for opening and monitoring the BBB of claim 14.
